# EUROPEAN PATENT APPLICATION

(11) **EP 3 721 914 A1**
(43) Date of publication of application: **14.10.2020**
(21) Application number: 18885324.6
(22) Date of filing: 03.12.2018
(51) Int. Cl.: A61L 9/03

(54) **AROMATHERAPY HEAD FIXING ASSEMBLY AND AROMATHERAPY APPARATUS**

(30) Priority: 04.12.2017 CN 201711254654
(71) Applicant: Hui, Ho Charm, Kwai Chung, Hong Kong 999077 (CN)
(72) Inventor: Hui, Ho Charm, Kwai Chung, Hong Kong 999077 (CN)
(74) Representative: Klunker IP Patentanwälte PartG mbB
(86) International application number: PCT/CN2018/118889
(87) International publication number: WO 2019/109882

(57) **Abstract**

Disclosed are an aroma diffuser head securing assembly and an aroma diffuser apparatus using the aroma diffuser head securing assembly, wherein the aroma diffuser head securing assembly is adapted for being connected with a neckless chamber body, the neckless chamber body having a receiving space and a chamber opening adapted for communicating the receiving space with an external environment, wherein the aroma diffuser head securing assembly including a top securing element, a middle securing element, and a bottom securing element, wherein the top securing element has a penetrative hole and is partially received in the chamber opening; the bottom securing element is received in the receiving space, a top end of the bottom securing element being connected to the top securing element and meanwhile abutting the middle securing element; and the middle securing element is sandwiched between the top securing element and the bottom securing element and is arranged to abut the chamber opening.

## Description

### FIELD

Embodiments of the present disclosure generally relate to the field of aroma diffusers, and more particularly relate to an aroma diffuser head securing assembly fitted with a neckless aroma diffuser chamber and an aroma diffuser apparatus using the aroma diffuser head securing assembly.

### BACKGROUND

An aroma diffuser apparatus comprises an aroma diffuser chamber and an aroma diffuser head assembly, wherein the aroma diffuser chamber is for holding an aroma essential oil and thus also referred to as an essential oil chamber. The aroma diffuser apparatus disclosed in the Chinese Utility Patent CN203525035U focuses on improving the structure of an aroma diffuser head assembly for the convenience of filling or replenishing an essential oil. However, like conventional aroma diffuser chambers, the aroma diffuser chamber of the aroma diffuser apparatus disclosed therein also adopts a necked container made of for example a glass material. Although a necked structure for mounting the aroma diffuser head assembly is easily formed with the glass material, it renders an undesired consumer experience as the glass material has a poor overall texture.

Colored glaze offers a special texture and an optimal use experience due to its profound handcraft tradition and cultural connotation. However, due to restrictions of the process for colored glaze formation, a top opening end formed needs to be polished into a planar structure; consequently, it is impossible to form a necked structure like a traditional aroma diffuser chamber. This is inconvenient for sealing the aroma diffuser chamber or directly mounting the aroma diffuser head assembly.

In other words, the colored glaze material can only produce a neckless aroma diffuser chamber. For the convenience of mounting the aroma diffuser head assembly, a specific necked structure may be glued to the chamber body of the aroma diffuser chamber made of the colored glaze material; however, due to the volatile properties of essential oil materials in a heated state and the specific chemical compositions thereof, the adhesive material is easily cured, which would otherwise cause sealing failure and finally cause the glued necked structure to be detached from the chamber body of the aroma diffuser apparatus, such that the aroma diffuser head assembly would become unusable. Therefore, for a neckless aroma diffuser chamber made of a colored glaze material, it is an urgent technical difficulty in the art to overcome to devise an aroma diffuser head securing assembly with an effective sealing and a secure connection.

### SUMMARY

A main objective of the present disclosure is to solve a technical problem that a chamber body of a neckless aroma diffuser chamber cannot be securely and firmly attached to an existing accessory structure such as an aroma diffuser head assembly.

According to a technical solution of the present disclosure, there is provided an aroma diffuser head securing assembly adapted for being fixedly connected to a neckless chamber body, the neckless chamber body having a receiving space and a chamber opening adapted for communicating the receiving space with an external environment, wherein the aroma diffuser head securing assembly comprises a top securing element, a middle securing element, and a bottom securing element, wherein the top securing element has a penetrative hole and is partially received in the chamber opening; the bottom securing element is received in the receiving space, a top end of the bottom securing element being connected to the top securing element and meanwhile abutting the middle securing element; and the middle securing element is sandwiched between the top securing element and the bottom securing element and is arranged to abut the chamber opening.

Preferably, the top securing element has a limiting boss, the limiting boss being configured for limiting the top securing element using a jig fitted with the limiting boss; the bottom securing element has a penetrative through- hole in which a limiting structure is provided; the jig fitted with the limiting structure may penetrate through the penetrative hole and the through-hole so as to be snap-fitted with the limiting structure and fasten the bottom securing element to the top securing element; meanwhile, the bottom securing element pushes open the middle securing element to abut the chamber opening.

Preferably, the limiting boss has multiple groups of oppositely disposed limiting portions, and axial rotation of the limiting boss is restricted by jigs fitted with the oppositely disposed limiting portions.

Preferably, the top securing element comprises a bottom boss, the bottom boss including a bottom boss sidewall and a bottom boss top plane, the bottom boss top plane being connected to the bottom boss sidewall, and the limiting boss being disposed on a surface of the bottom boss.

Preferably, the middle securing element comprises a body portion and a snap sheet, a through-hole being arranged at a middle part of the body portion along an axial direction; the snap sheet is provided in plurality, the plurality of snap sheets being evenly distributed at a through-hole edge at a bottom surface of the body portion and connected to the through-hole edge; and snap-fit structures are arranged axially in inner surfaces of free ends of the plurality of snap sheets.

Preferably, the aroma diffuser head securing assembly further comprises a top sealing ring and a bottom sealing ring; a top face and the bottom face of the body portion are provided with a top face recessed groove and a bottom face recessed groove which are arranged surrounding the through-hole, respectively; and the top sealing ring and the bottom sealing ring are provided in the top face recessed groove and the bottom face recessed groove of the body portion, respectively.

Preferably, the top securing element comprises a bottom connecting tube, the bottom connecting tube penetrating through the through-hole of the body portion, and a bottom end of the bottom connecting tube and a top end of the bottom securing element are thread connected.

Preferably, a bevel structure is provided at the top end of the bottom securing element, along an axial direction of the bevel structure being arranged snap-fit structures; the snap-fit structures on the bevel structure are arranged to fit with the snap-fit structures of the snap sheets; and the bevel structure is adapted for pushing open the plurality of snap sheets so as to securely abut the chamber opening.

According to another technical solution of the present disclosure, an aroma diffuser apparatus is provided, comprising: a neckless chamber body, an aroma diffuser head securing assembly, a burner head assembly, a burner head lid, and a protective cover, wherein the burner head assembly is partially disposed at a top portion of the penetrative hole and partially extends till inside the receiving space; the burner head lid is configured for covering the burner head assembly; the protective cover is a hollow-carved structure to cover the top portion of the neckless chamber body; and the aroma diffuser head securing assembly is the one mentioned above.

Preferably, the neckless chamber body comprises a chamber body platform and a chamber opening disposed on the chamber body platform.

The chamber body of the diffuser apparatus provided by the present disclosure is a neckless chamber body; the aroma diffuser head securing assembly may be firmly attached to the chamber body, thereby providing advantages such as a tight and firm connection and a stable structure.

### BRIEF DESCRIPTION OF THE DRAWINGS

To elucidate the technical solutions of the present disclosure, the drawings used for describing the embodiments will be briefly introduced below. It is apparent that the drawings as described only relate to some embodiments of the present disclosure. To those skilled in the art, other drawings may be derived based on these drawings without exercise of inventive work, wherein:
Fig. 1A is an exploded structural schematic diagram from a perspective view of an aroma diffuser apparatus provided by the present disclosure;
Fig. 1B is an exploded structural schematic diagram from a planar view of an aroma diffuser apparatus provided by the present disclosure;
Fig. 2A is a semi-sectional view of the aroma diffuser apparatus in an assembled state according to the present disclosure;
Fig. 2B shows a full-sectional view of the aroma diffuser apparatus in an assembled state according to the present disclosure;
Fig. 3 shows a schematic diagram of a neckless chamber body structure of an aroma diffuser apparatus according to the present disclosure;
Fig. 4 shows a structural schematic diagram of a top securing element of an aroma diffuser apparatus according to the present disclosure;
Fig. 5 shows a structural schematic diagram from another view of a top securing element of an aroma diffuser apparatus shown in Fig. 4;
Fig. 6 shows a structural schematic diagram of a middle securing element of an aroma diffuser apparatus according to the present disclosure; and
Fig. 7 shows a partially structural schematic diagram of a middle securing element shown in Fig. 6.

### List of Reference Numerals:

aroma diffuser apparatus 100; neckless chamber body 10; chamber body platform 11; chamber opening 12; aroma diffuser head securing assembly 20; top securing element 21; middle securing element 22; bottom securing element 23; body portion 221; top sealing ring 222; bottom sealing ring 223; snap sheet 224; burner head assembly 30; burner head lid 40; protective cover 50.

### DETAILED DESCRIPTION OF EMBODIMENTS

Hereinafter, the technical solutions in the embodiments of the present disclosure will be described in a clear and complete manner; apparently, the embodiments described are only part of the embodiments of the present disclosure, not all of them. All other embodiments obtained by those skilled in the art without exercise of inventive work based on the embodiments in the present disclosure all fall within the protection scope of the present disclosure.

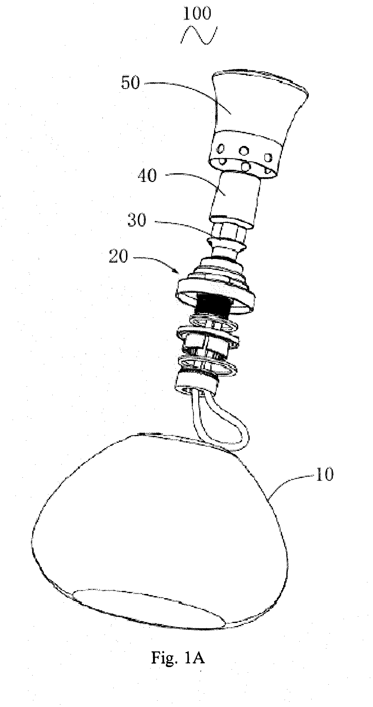

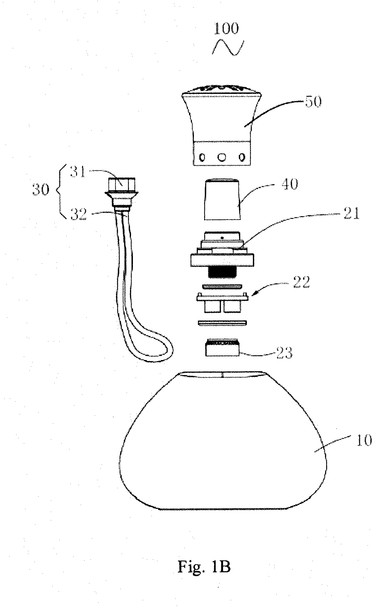

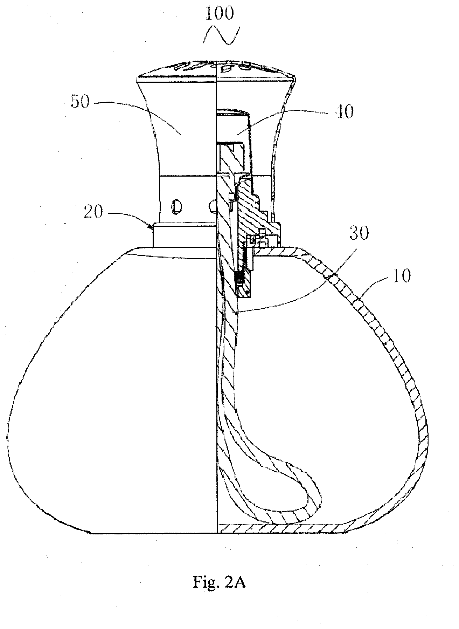

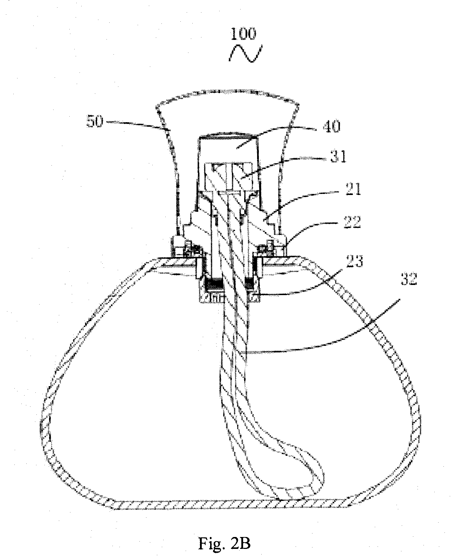

Please refer to Fig. 1A, Fig. 1B, Fig. 2A, and Fig. 2B simultaneously, wherein Fig. 1A and Fig. 1B are exploded status diagrams of an aroma diffuser apparatus according to a preferred embodiment of the present disclosure; and Fig. 2A and Fig. 2B are assembled status diagrams of the aroma diffuser apparatus according to the preferred embodiment of the present disclosure. As shown in Fig. 1A and Fig. 2A, the aroma diffuser apparatus 100 generally comprises a neckless chamber body 10, an aroma diffuser head securing assembly 20, a burner head assembly 30, a burner head lid 40, and a protective cover 50. Additionally, as shown in Fig. 1B and Fig. 2B, the aroma diffuser head securing assembly 20 further comprises a top securing element 21; a middle securing element 22; and a bottom securing element 23. The burner head assembly 30 further comprises a ceramic burner head 31 and a wick 32. Here, it needs to be particularly noted that the neckless chamber body 10 shown in the figure is prominently different from a conventional aroma diffuser chamber with a necked structure; correspondingly, another improvement of the present disclosure lies in the aroma diffuser head securing assembly 20 which is fixedly mounted on the neckless chamber body 10 and is fitted therewith to form a necked structure. Hereinafter, details of the aroma diffuser head securing assembly 20 will be described with reference to the accompanying drawings, while structures of other components not directly related to the present disclosure will not be described.

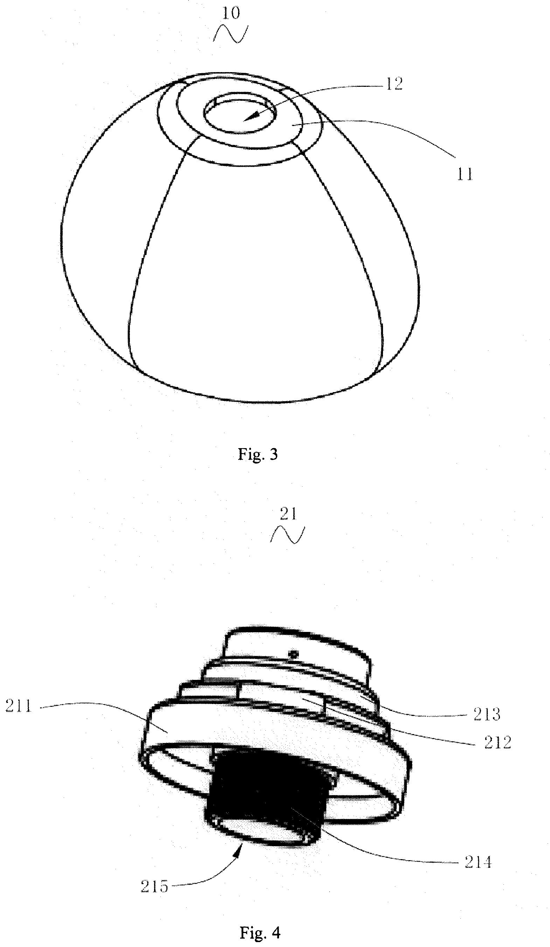

Please further refer to Fig. 3, where the neckless chamber body 10 has a receiving space inside, the receiving space being adapted for receiving an aroma diffusion-purpose liquid substance, e.g., alcohol, essential oil, or other liquids. In this embodiment, the neckless chamber body 10 comprises a chamber body platform 11; and a chamber opening 12 disposed at a central portion of the chamber body platform 11, wherein the chamber body 11 is disposed at a top portion of the neckless chamber body 10. In this embodiment, the chamber body platform 11 is preferably a planar structure. Of course, the structure of the chamber body platform 11 may also be adjusted correspondingly based on actual needs. However, for the purpose of effective sealing, at least part of the area of the chamber body platform 11 is a planar structure for the purpose of mounting the aroma diffuser head securing assembly 20 securely.

As shown in Fig. 3, the chamber opening 12 is disposed on the chamber body platform 11. More accurately, the chamber opening 12 is disposed at a central portion of the planar structure of the chamber body platform 11. Of course, in other embodiments, a position of the chamber opening 12 on the chamber body platform 11 may be adjusted according to actual needs, which will not be limited herein. Preferably, the chamber opening 12 is a round chamber opening. The chamber opening 12 communicates with the receiving space of the neckless chamber body 10. In other words, the chamber opening 12 may communicate the receiving space with an external environment.

Hereinafter, relevant details of the aroma diffuser head securing assembly 20 will be illustrated with reference to the accompanying drawings. The aroma diffuser head securing assembly 20 is securely connected to the neckless chamber body 10, forming a terraced necked structured of the neckless chamber body 10 so as to bear, on the aroma diffuser head securing assembly 20, the burner head assembly 30, the burner head lid 40, and the protective cover 50. Specifically, the aroma diffuser head securing assembly 20 is securely disposed on the chamber opening 12, facilitating a user to charge an aroma liquid into the neckless chamber body 10 for storage or use. As shown in Fig. 2B, in an assembled state, the middle securing element 22 is disposed between the top securing element 21 and the bottom securing element 23; and the bottom securing element 23 is thread connected to the top securing element 21. Hereinafter, the top securing element 21, the middle securing element 22, and the bottom securing element 23 forming the aroma diffuser head securing assembly 20 will be illustrated, respectively.

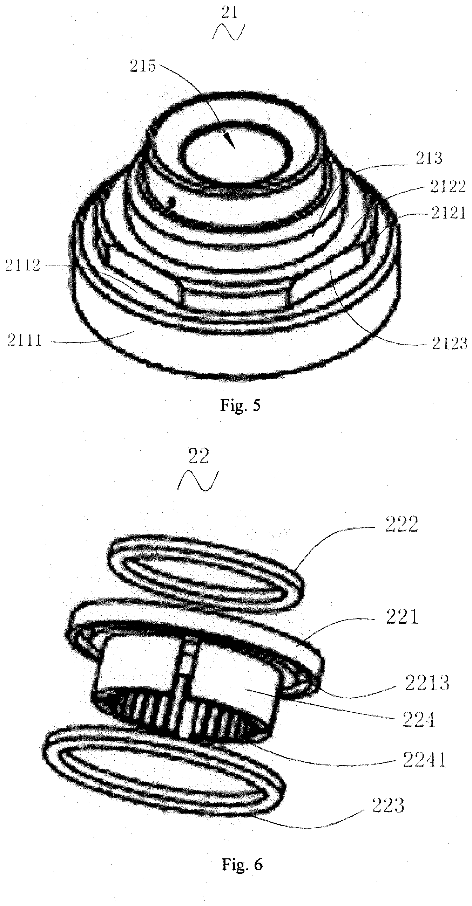

First, the structural details of the top securing element 21 will be illustrated with reference to Fig. 4 and Fig. 5. The top securing element 21 comprises: a bottom boss 211 which may bear the protective cover 50; a limiting boss 212; a top boss 213 which may bear the burner head lid 40; and a bottom connecting tube 214 for connecting the bottom securing element 23. Additionally, the top securing element 21 further has a penetrative hole 215 that penetrates axially. In this embodiment, the top securing element 21 is preferably an integrally machined structure, and the top securing element 21 is preferably made of a metal aluminum material.

Further, the bottom boss 211 which generally has a lid shape comprises a bottom boss sidewall 2111 and a bottom boss top plane 2112; in a service state, the protective cover 50 may be disposed, in a freely removable manner, on the bottom boss top plane 2112; in an assembled state, the bottom boss sidewall 2111 is non-detachably abutting the planar structure of the chamber body platform 11 of the bottom opening 12. In this embodiment, the bottom boss 211 has a cylindrical shape. Of course, in other embodiments, the bottom boss 211 may have a structure of other shape, which may be correspondingly adjusted according to actual needs, and the present disclosure has no limitation thereto.

The limiting boss 212 is disposed, in a terraced manner, on a top plane of the bottom boss top plane 2112 and is joined to the bottom boss top plane 2112. Preferably, the limiting boss 212 and the bottom boss 211 are coaxially arranged. In this embodiment, the limiting boss 212 and the bottom boss 211 are integrally structured. The limiting boss 212 comprises a limiting boss sidewall 2121 and a limiting boss top plane 2122. The limiting boss 212 generally has a prism shape, and the limiting convex sidewall 2121 has a plurality of limiting portions 2123 for limiting the top securing element 21. Preferably, the limiting portion 2123 is provided in two or four, wherein the plurality of limiting portions 2123 are symmetrically arranged. The limiting portion 2123 may facilitate the user to limit the top securing element 21 so as to be capable of conveniently assembling the aroma diffuser head securing assembly 20 to the neckless chamber body 10. Specifically, when it is needed to limit the limiting boss 212, the limiting boss 212 is clamped to the limiting portion 2123 via a fitted clamping jig, thereby restricting the limiting boss 212 to rotate axially, so as to further restrict axial rotation of the top securing element 21.

The top boss 213 is further disposed, in a terraced manner, on the limiting boss top plane 2122. The top boss 213 generally has a cylindrical shape, configurable for bearing the burner head lid 40, and the top boss 213 and the limiting boss 212 are integrally structured. Additionally, the top boss 213 and the limiting boss 212 are coaxially arranged.

The bottom connecting tube 214 is disposed on a bottom face of the bottom boss top plane 2112 and is also arranged coaxially with the bottom boss top plane 2112. In other words, the bottom connecting tube 214 and the bottom boss 211 are of a coaxial structure, and the limiting boss 212 and the top boss 213 are also of a coaxial structure. A thread structure is disposed at an outer side of the bottom connecting tube 214.

The penetrative hole 215 simultaneously penetrates through the bottom boss 211, the limiting boss 212, the top boss 213, and the bottom connecting tube 214. Preferably, the penetrative hole 215 is arranged coaxially with the bottom boss 211, the limiting boss 212, the top boss 213, and the bottom connecting tube 214.

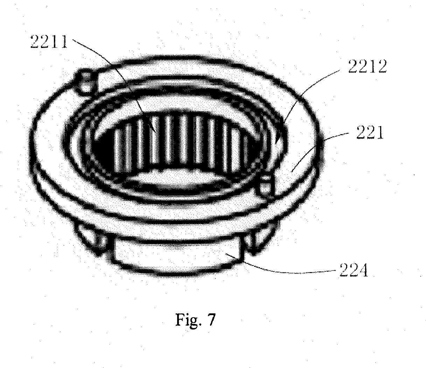

Next, structural details of the middle securing element 22 will be illustrated with reference to Fig. 6 and Fig. 7. The middle securing element 22 comprises a body portion 221, a top sealing ring 222, a bottom sealing ring 223, and a plurality of snap sheets 224, wherein the plurality of snap sheets 224 are disposed at the bottom of the body portion 221; and preferably, two positioning pins are arranged at the top of the body portion 221 so as to facilitate two corresponding positioning recesses to be mutually positioned on the bottom of the bottom boss 211 of the top securing element 21 during assembling. Preferably, the body portion 221 and the plurality of snap sheets 224 of the middle securing element 22 are made of a plastic material.

An axially arranged through-hole 2211 is provided in the middle part of the body portion 221. When the aroma diffuser apparatus 100 is in an assembled state, the bottom connecting tube 214 is disposed within the through- hole 2211 of the body portion 221. A top surface of the body portion 221 has a top face recessed groove 2212 arranged surrounding the through-hole 2211, and the top face recessed groove 2212 is disposed proximal to an edge of the through-hole 2211. Additionally, a bottom surface of the body portion 221 has a bottom face recessed groove 2213 arranged surrounding the through-hole 2211, and the bottom face recessed groove 2213 is disposed proximal to an edge of the bottom surface of the body portion 221. In this embodiment, the body portion 221 and the snap sheets 224 are integrally structured.

The top sealing ring 222 is disposed in the top face recessed groove 2212, and the bottom sealing ring 223 is disposed in the bottom face recessed groove 2213. It may be understood that the top sealing ring 222 and the bottom sealing ring 223 are fitted with the top recessed groove 2212 and the bottom recessed groove 2213, respectively, to jointly implement a reliable sealing of the aroma diffuser apparatus 100. Specifically, when the aroma diffuser apparatus 100 is in an assembled state, the top face sealing ring 222 disposed in the top face recessed groove 2212 abuts the top securing element 21; a bottom face sealing ring 222 disposed in the bottom face recessed groove 2213 abuts the chamber body platform 11 to thereby seal the through-hole of the neckless chamber body 10 so as to prevent the liquid in the neckless chamber body 10 from being diffused out of the through-hole edge. Preferably, the top sealing ring 222 and the bottom sealing ring 223 are made of a rubber material.

One end of the snap sheet 224 is connected to a bottom surface of the body portion 221, and the other end thereof is a free end. Specifically, the snap sheet 224 is arranged along an edge of the through-hole 2211. The snap sheet 224 is provided in plurality, the plurality of snap sheets 224 are evenly arranged along the edge of the through-hole 2211. Gaps exist between the plurality of snap sheets 224. The gaps between the plurality of snap sheets 224 allow the snap sheets 224 to have a very large deformation tolerance to prevent deformation caused by thermal expansion. A diameter of the cylindrical body enclosed by the plurality of snap sheets 224 is slightly smaller than a diameter of the chamber opening 12 such that the plurality of snap sheets 224 may be partially received in the chamber opening 12, and free ends of the plurality of snap sheets 224 may penetrate through the chamber opening 12 and extend till inside the neckless chamber body 10. It is additionally noted that although the outer portions of the plurality of snap sheets 224 enclose a cylindrical body, the present disclosure is not limited thereto; for example, if an irregular shape (e.g., a polygonal column) is adopted inside the chamber opening 12, the external shape of the plurality of snap sheets 224 adopts a polygonal column shape fitted thereto.

In this embodiment, the snap sheet 224 is provided in four, the four snap sheets 224 being evenly arranged along the edge of the through-hole 2211; however, the present disclosure is not limited thereto. It may be understood that in other embodiments, the snap sheets 224 may be provided in other numbers, which is not limited herein. Additionally, a plurality of serrated snap-joints 2241 are arranged axially at free ends of the snap sheets 224. The function of snap-joints 2241 will be illustrated below.

Finally, the bottom securing element 23 connected respectively to the top securing element 21 and the middle securing element 22 will be illustrated with reference to Fig. 1B land Fig. 2B, where the bottom securing element 23 likewise has a through-hole structure disposed axially penetrating through the bottom securing element 23. A top end of the bottom securing element 23 is connected to a tip end of the bottom connecting tube 214 of the top securing element 20. It may be understood that an inner threshold structure is disposed inside a top end of the through-hole of the bottom securing element 23 and is fitted with an external threshold structure of the bottom connecting tube 214, thereby implementing a thread connection between the bottom securing element 23 and the top securing element 21. Additionally, a bevel structure is further arranged external to the top end of the bottom securing element 23, i.e., the bevel structure is disposed at an outer edge of one end of the bottom securing element 23 connected to the bottom connecting tube 214, and a plurality of serrated snap-fit structures are also arranged axially on the surface of the bevel structure and are evenly distributed along an outer portion of the top end of the bottom securing element 23. The snap-fit structures on the surface of the bevel structure are fitted with the snap-joints 2241 of the snap 2 sheets 24, which together implement a function of preventing detachment of the bottom securing element 23 from the top securing element 21 and the middle securing element 22.

Further, a polygonal structure is provided inside the bottom end of the through-hole of the bottom securing element 23, which facilitates a user to use a specific jig that extends into the through-hole of the bottom securing element 23 to be fitted with the bottom end of the bottom securing element 23, and by rotating the bottom securing element 23, an inner thread of the through-hole of the bottom securing element 23 is engaged with an outer thread of the bottom connecting tube 214 of the top securing element 21; meanwhile, snap-fit structures at the outer portion of the top end of the bottom securing element 23 are snapped-on with the plurality of snap-joints 2241 of the snap sheets 224, such that the bottom securing element 23 further abuts the snap sheets 224 of the middle securing element 22 to realize the anti-detachment function. Preferably, the bottom securing element 23 is made of a metal material.

To understand the connection manner between the neckless chamber body 10 and the aroma diffuser head securing assembly 20 according to the embodiments of the present disclosure more clearly, a method of assembling the two will be described as a whole.

First, pre-assembling the aroma diffuser head securing assembly 20;
wherein the body portion 221, the top sealing ring 222, and the bottom sealing ring 223 of the middle securing element 22 are assembled together; and the assembled middle securing element 22 is pre-assembled with the top securing element 21 and the bottom securing element 23. At this point, the middle securing element 22 is sandwiched between the top securing element 21 and the bottom securing element 23. The bottom securing element 23 is thread connected to the bottom connecting tube 214 of the top securing element 21. It may be understood that at this point, the bottom securing element 23 and the bottom connecting tube 214 are only preliminarily connected, i.e., they are only preliminarily thread connected, rather than finally securing.

Then, disposing the aroma diffuser head securing assembly 20 inside the chamber opening 12 of the neckless chamber body 10.

At this point, the bottom securing element 23 is disposed inside the receiving space of the neckless chamber body 10, the snap sheets 224 of the middle securing element 22 penetrate through the chamber opening 12, and free ends of the snap sheets 224 are received in the receiving space.

Next, further securing the aroma diffuser head securing assembly 20 to the neckless chamber body 10.

Upon further securing the aroma diffuser head securing assembly 20 to the neckless chamber body 10, a specific jig is adopted to securely limit the limiting boss 212 of the top securing element 21; meanwhile, another specific jig is inserted from the penetrative hole 215 to be snap-fitted with a polygonal structure of the bottom securing element 23, thereby facilitating the jig to rotate the bottom securing element 23 from the outside. During the process of rotating the bottom securing element 23, the bottom securing element 23 moves towards the direction of the top securing element 21; and at this point, the bevel structure of the bottom securing element 23 will gradually abut the snap sheets 224 of the middle securing element 22 and gradually extrude the snap sheets 224 to the outside. In other words, at this point, free ends of the snap sheets 224 will be pushed open during the process of rotating the bottom securing element 23, i.e., moving along a direction reverse to its medial axis. At this point, the free ends of the snap sheets 224 will firmly abut the chamber opening 12, so as to be capable of securely connecting the aroma diffuser head securing assembly 20 to the neckless chamber body 10. Further, the snap-fit structures on the bevel structure of the bottom securing element 23 may be mutually snap-fitted to a plurality of snap-joints 2241 on the snap sheets 224, which may prevent loosening of the bottom securing element 23, causing the aroma diffuser head securing assembly 20 to be securely and firmly connected with the neckless chamber body 10.

Additionally, the burner head assembly 30 comprises a ceramic burner head 31 and a wick 32. The ceramic burner head 31 is connected to the wick 32. The ceramic burner head 31 may be inserted into the penetrative hole 215 of the top boss 213. The ceramic burner head 31 is connected to the wick 32 and may receive a combustible liquid delivered by the wick 32. In the present disclosure, the ceramic burner head 31 is made of a high temperature-resistance material, e.g., ceramic, etc. One end of the wick 32 is connected to the ceramic burner head 31, and the other end thereof extends from the penetrative hole 215 into the receiving space of the neckless chamber body 10.

The burner head lid 40 may cover the top boss 213. In this embodiment, the burner head lid 40 is fitted with the top boss 213 such that it may exactly cover the top boss 213. The burner head lid 40 may be adapted for covering and extinguishing the flame, and meanwhile may play a role of sealing the liquid inside the neckless chamber body 10, preventing volatility of the liquid inside the receiving space.

The protective cover 50 is a hollow-carved structure, for covering the top securing element 21, and a bottom face of the protective cover 50 abuts the bottom boss 211, wherein the protective cover 50 may prevent scalding the user. It is easily understood that the protective cover 50 may be directly carried on the chamber body platform 11 of the neckless chamber body 10.

The neckless chamber body 10 of the aroma diffuser apparatus 100 provided by the present disclosure is a neckless container; the aroma diffuser head securing assembly 20 may be firmly attached to the chamber body 10, and has advantages such as a tight and firm connection and a stable structure. It may be understood that the aroma diffuser head securing assembly 20 in the present disclosure may be easily adapted to a chamber body of a neckless chamber body of other type or for other purposes, and the present disclosure has no limitation thereto.

What have been described above are only embodiments of the present disclosure, not for limiting the patent scope of the present disclosure. Any equivalent structure or equivalent flow change made based on the contents in the specification of the present disclosure, or a direct or indirect application to other related technical fields, should be included in the protection scope of the present disclosure.

## Claims

1. An aroma diffuser head securing assembly (20) adapted for being fixedly connected with a neckless chamber body (10), the neckless chamber body (10) having a receiving space and a chamber opening (12) which is adapted for communicating the receiving space with an external environment, wherein the aroma diffuser head securing assembly (20) comprises:
a top securing element (21);
a middle securing element (22); and
a bottom securing element (23); wherein
the top securing element (21) has a penetrative hole (215) and is partially received in the chamber opening (12);
the bottom securing element (23) is received in the receiving space, a top end of the bottom securing element (23) being connected to the top securing element (21) and meanwhile abutting the middle securing element (22); and
the middle securing element (22) is sandwiched between the top securing element (21) and the bottom securing element (23) and is arranged to abut the chamber opening (12).

2. The aroma diffuser head securing assembly (20) according to claim 1, wherein the top securing element (21) has a limiting boss (212), the limiting boss (212) being configured for limiting the top securing element (21) using a jig fitted with the limiting boss (212); the bottom securing element (23) has a penetrative through- hole in which a limiting structure is provided; the jig fitted with the limiting structure may penetrate through the penetrative hole (215) and the through-hole so as to be snap-fitted with the limiting structure and fasten the bottom securing element (23) to the top securing element (21); meanwhile, the bottom securing element (23) pushes open the middle securing element (22) to abut the chamber opening (12).

3. The aroma diffuser head securing assembly (20) according to claim 2, wherein the limiting boss (212) has multiple groups of oppositely disposed limiting portions (2123), and axial rotation of the limiting boss (212) is restricted by jigs fitted with the oppositely disposed limiting portions (2123).

4. The aroma diffuser head securing assembly (20) according to claim 2, wherein the top securing element (21) comprises a bottom boss (211), the bottom boss (211) including a bottom boss sidewall (2111) and a bottom boss top plane (2112), the bottom boss top plane (2112) being connected to the bottom boss sidewall (2111), and the limiting boss (212) being disposed on a surface of the bottom boss (211).

5. The aroma diffuser head securing assembly (20) according to claim 2, wherein the middle securing element (22) comprises a body portion (221) and a snap sheet (224), a through-hole being arranged at a middle part of the body portion along an axial direction; the snap sheet (224) is provided in plurality, the plurality of snap sheets (224) being evenly distributed at an edge of the through-hole at a bottom surface of the body portion (221) and connected to the edge of the through-hole; and snap-fit structures are arranged axially in inner surfaces of free ends of the plurality of snap sheets (224).

6. The aroma diffuser head securing assembly (20) according to claim 5, further comprising a top sealing ring (222) and a bottom sealing ring (223); wherein a top face and the bottom face of the body portion (221) are provided with a top face recessed groove (2212) and a bottom face recessed groove (2213) which are arranged surrounding the through-hole, respectively; and the top sealing ring (222) and the bottom sealing ring (223) are provided in the top face recessed groove (2212) and the bottom face recessed groove (2213) of the body portion (221), respectively.

7. The aroma diffuser head securing assembly (20) according to claim 5, wherein the top securing element (21) comprises a bottom connecting tube (214), the bottom connecting tube (214) penetrating through the through-hole of the body portion, and a bottom end of the bottom connecting tube (214) and a top end of the bottom securing element (23) are thread connected.

8. The aroma diffuser head securing assembly (20) according to claim 5, wherein a bevel structure is provided at the top end of the bottom securing element (23), along an axial direction of the bevel structure being arranged snap-fit structures; the snap-fit structures on the bevel structure are arranged to fit with the snap-fit structures of the snap sheets (224); and the bevel structure is adapted for pushing open the plurality of snap sheets (224) so as to securely abut the chamber opening (12).

9. An aroma diffuser apparatus (100), comprising:
a neckless chamber body (10);
an aroma diffuser head securing assembly (20);
a burner head assembly (30);
a burner head lid (40); and
a protective cover (50);
wherein the burner head assembly (30) is partially disposed at a top portion of the penetrative hole (215) and partially extends till inside the receiving space;
the burner head lid (40) is configured for covering the burner head assembly (30);
the protective cover (50) is a hollow-carved structure to cover the top portion of the neckless chamber body (10); and
the aroma diffuser head securing assembly (20) is the one according to any of claims 1-8.

10. The aroma diffuser apparatus (100) according to claim 9, wherein the neckless chamber body comprises: a chamber body platform (11); and a chamber opening (12) disposed on the chamber body platform.
